# EUROPEAN PATENT SPECIFICATION

(11) **EP 4 649 887 B1**
(45) Date of publication and mention of the grant of the patent: **21.10.2026**
(21) Application number: 24191551.1
(22) Date of filing: 29.07.2024
(51) Int. Cl.: A61B 5/11

(54) **MOBILE ROBOT FOR GAIT ANALYSIS**
MOBILER ROBOTER ZUR GANGANALYSE
ROBOT MOBILE POUR ANALYSE DE DÉMARCHE

(30) Priority: 17.05.2024 KR 20240064844
(43) Date of publication of application: 19.11.2025
(73) Proprietor: Medical Solution System, Gimhae city, Gyeongsangnam-do 50834 (KR)
(72) Inventor: Kang, Minsoo, 47507 Busan (KR); Seo, JoonMo, 48270 Busan (KR)
(74) Representative: M. Zardi & Co S.A.

(56) References cited:
- CN-A- 111 603 171
- CN-A- 111 603 172
- CN-A- 113 229 809
- KR-B1- 102 250 176
- US-A1- 2022 241 970
- US-A1- 2022 331 028
- DUONG HUU TOAN ET AL: "Human Gait Estimation Using Multiple 2D LiDARs", IEEE ACCESS, IEEE, USA, vol. 9, 5 April 2021 (2021-04-05), pages 56881 - 56892, XP011849906, DOI: 10.1109/ACCESS.2021.3071147
- ZHU FEI ET AL: "Extrinsic Calibration of Multiple Two-Dimensional Laser Rangefinders Based on a Trihedron", SENSORS, vol. 20, no. 7, 26 March 2020 (2020-03-26), CH, pages 1837, XP055927133, ISSN: 1424-8220, DOI: 10.3390/s20071837

## Description

### CROSS REFERENCE TO RELATED APPLICATION

The present application claims priority to Korean Patent Application No. 10-2024-0064844, filed on May 17, 2024.

### BACKGROUND

### Technical Field

The present disclosure relates to a mobile robot for gait analysis and, more particularly, to a mobile robot for gait analysis, which analyzes the gait of a subject to be measured.

### Description of the Related Art

The description in this section merely provides background information on an exemplary embodiment of the present disclosure and does not constitute the prior art.

Gait analysis is to find out the singularity of the walking pattern, and performs a comprehensive evaluation by measuring various aspects of gait. More specifically, it may include kinematic analysis, dynamic electromyography, and energy expenditure measurement. This gait analysis can be applied to identify the posture or movement characteristics of people with lower extremity lesions and spinal cord misalignments, or to help athletes run more efficiently.

Currently, gait analysis in rehabilitation medicine measures and analyzes gait by using expensive load-measuring mattresses or high-speed cameras. However, this type of gait measurement has problems such as requiring various sensors to be attached to the subject's body, being expensive, and taking a long time to prepare for the measurement. In addition, there are limitations in analyzing an everyday gait since the analysis is performed in a controlled environment such as a laboratory.

Recently, as people spend more time sitting, scoliosis, pelvic distortion, and flat feet have become more common, and these problems affect gait. In particular, for children and adolescents, it is important to detect and correct the wrong posture early and ensure development of good walking habits.

Although the need for gait measurement and analysis is increasing, conventional gait analysis methods are not easily accessible due to the cost and limitations. Therefore, it is necessary to develop a technology that can analyze gait more simply and at a lower cost.

Meanwhile, as prior art related to the present disclosure, a Korean patent has been disclosed (Patent No. 10-1895399, "Gait analysis and correction apparatus base of information and communications technology", Registration Date: August 30, 2018).

The background technology described above is technical information that the inventor possessed for deriving the present disclosure or acquired in the process of deriving the present disclosure and cannot necessarily be said to be known to the general public before filing the application for the present disclosure.

US2022331028A1 discloses a method that comprises capturing a plurality of images of a person executing a movement sequence by means of a contactless sensor, the plurality of images representing the movements of the person's body elements; generating at least one skeleton model having limb positions for at least some of the plurality of images; and calculating a movement pattern from the movements of the body elements by comparing changes in the limb positions in the generated skeleton model. In addition, vital signs and/or signal processing parameters of the person can be acquired and evaluated. US2022241970 discloses a robot that includes a sensor, a motor, and a processor configured to identify a user based on data obtained through the sensor; identify a position among a plurality of positions located at a preset distance from the user based on the user's position and movement direction; control the motor to move the robot to the identified position; and obtain gait data corresponding to the user based on the data obtained through the sensor while tracking the user's legs.

### SUMMARY

The present disclosure is proposed to solve the above-mentioned problems of the previously proposed methods, and aims to provide a mobile robot for gait analysis according to claim 1 that performs gait analysis simply and easily without attaching a sensor to the body of the subject to be measured and that continuously analyzes gait without restrictions on places since tracking and measuring the subject to be measured, by measuring the movement of the feet of the subject to be measured and by analyzing the gait using the scanning information of a gait measurement LiDAR sensor unit installed to protrude forward from the side of the main body while following the subject to be measured with a preset distance maintained using the scanning information of a front LiDAR sensor unit mounted on the main body.

However, the technical task to be achieved by the present disclosure is not limited to the technical task described above, other technical tasks may exist, and even if not explicitly mentioned, it also includes purposes or effects that can be identified from the means of solving the tasks or the forms of implementation.

A mobile robot for gait analysis according to the characteristics of the present disclosure for achieving the above objective includes a main body; a driving unit for moving the main body; a front LiDAR sensor unit being mounted on the main body and scanning a front side; a controller for controlling the driving unit to follow a subject to be measured with a preset distance maintained by using scanning information of the front LiDAR sensor unit; a gait measurement LiDAR sensor unit being installed to protrude forward from a side of the main body and measuring movements of feet of the subject to be measured; and a gait analysis unit for analyzing a gait of the subject to be measured by using the movements of the feet measured by the gait measurement LiDAR sensor unit, wherein the gait measurement LiDAR sensor unit measures the movements of the feet including changes in position, direction, and shape of left and right feet by scanning a plane at a foot height of the subject to be measured, and characterized in that the gait measurement LiDAR sensor unit comprises a left LiDAR sensor unit being installed to protrude forward from a left side of the main body and measuring the movement of a left foot of the subject to be measured, and a right LiDAR sensor unit being installed to protrude forward from a right side of the main body and measuring the movement of a right foot of the subject to be measured.

According to the mobile robot for gait analysis proposed in the present disclosure, gait analysis is simply and easily performed without attaching a sensor to the body of the subject to be measured and the gait is continuously analyzed without restrictions on places since tracking and measuring the subject to be measured, by measuring the movement of the foot of the subject to be measured and by analyzing the gait using the scanning information of the gait measurement LiDAR sensor unit installed to protrude forward from the side of the main body while following the subject to be measured with a preset distance maintained.

In addition, various and beneficial advantages and effects of the present disclosure are not limited to the content described above, and may be more easily understood in the process of explaining specific exemplary embodiments of the present disclosure.

### BRIEF DESCRIPTION OF THE DRAWINGS

FIG. 1 is a view showing a configuration of a mobile robot for gait analysis according to an exemplary embodiment of the present disclosure.
FIG. 2 is a view showing the overall appearance of a mobile robot for gait analysis according to an exemplary embodiment of the present disclosure.
FIG. 3 is a top view of a mobile robot for gait analysis according to an exemplary embodiment of the present disclosure.
FIG. 4 is a view showing that a mobile robot for gait analysis moves and scans the gait of a subject to be measured according to an exemplary embodiment of the present disclosure.
FIG. 5 is a view showing that a gait measurement LiDAR sensor unit of a mobile robot for gait analysis measures the movement of the foot of a subject to be measured according to an exemplary embodiment of the present disclosure.
FIG. 6 is a view showing that a gait measurement LiDAR sensor unit of a mobile robot for gait analysis includes a first LiDAR sensor unit and a second LiDAR sensor unit according to an exemplary embodiment of the present disclosure.

### DETAILED DESCRIPTION OF THE DISCLOSURE

With reference to the attached drawings below, exemplary embodiments of the present disclosure will be described in detail so that those of ordinary skill in the technical field to which the present disclosure belongs may easily implement the present disclosure. However, the present disclosure may be implemented in various forms, and is not limited to the exemplary embodiments described herein. Also, in order to clearly describe the present disclosure in the drawings, parts irrelevant to the description are omitted, and similar parts are given similar reference numerals throughout the specification.

Throughout the specification, when an element is "connected" to another element, it includes not only the case where it is "directly connected" but also the case where it is "indirectly connected" to another element with any element interposed in between. In addition, terms such as "include" "provided" or "have" described below should be construed as trying to specify that a feature, number, step, motion, component, part or combination thereof exists in the specification, and it should be understood that the existence or additional possibilities of one or more other features, numbers, steps, motions, components, parts, or combinations thereof are not excluded in advance. In addition, singular expressions used in the present disclosure include plural expressions unless the context clearly indicates otherwise.

In addition, each configuration, process, procedure, method, etc. included in each exemplary embodiment of the present disclosure may be shared within a range that does not technically contradict each other.

In addition, terms such as "...portion", "...device", "...module", and the like used in the specification refer to a unit that processes at least one function or operation, which may be implemented as hardware or software or a combination of hardware and software.

In addition, some of the operations or functions described as being performed by the terminal, apparatus, or device in the present disclosure may be performed by a server connected to the terminal, apparatus, or device. Likewise, some of the operations or functions described as being performed by the server may also be performed by the terminal, apparatus, or device connected to the server.

In particular, a means for executing a system according to each exemplary embodiment of the present disclosure may be an application or a web server, and a terminal as the means that reads a recording medium in which the application or web server is recorded may include not only general PCs such as desktops and laptops, but also mobile terminals such as smartphones and tablet PCs.

The following exemplary embodiments are detailed explanations for assisting in the understanding of the present disclosure and do not limit the scope of the present disclosure. Therefore, inventions performing the same function at the same scope as the present disclosure will also fall within the scope of rights of the present disclosure.

Hereinafter, exemplary embodiments of the present disclosure will be described in detail with reference to the accompanying drawings.

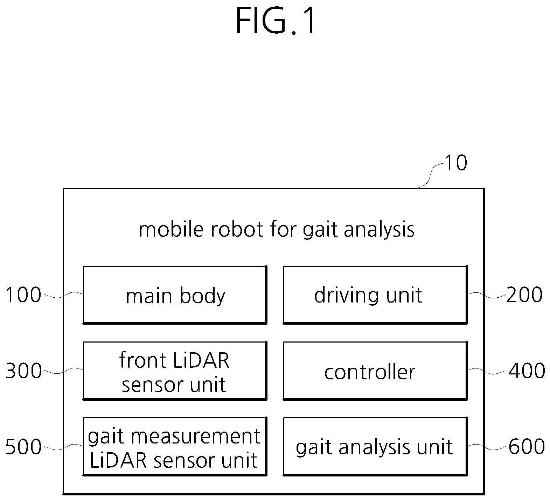

FIG. 1 is a view showing a configuration of a mobile robot 10 for gait analysis according to an exemplary embodiment of the present disclosure. As shown in FIG. 1, the mobile robot 10 for gait analysis according to an exemplary embodiment of the present disclosure may include a main body 100, a driving unit 200, a front LiDAR sensor unit 300, a controller 400, a gait measurement LiDAR sensor unit 500, and a gait analysis unit 600.

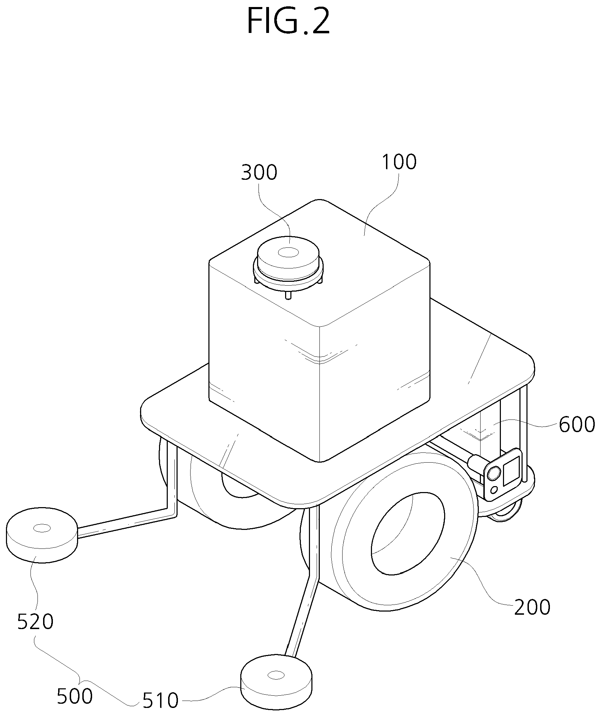

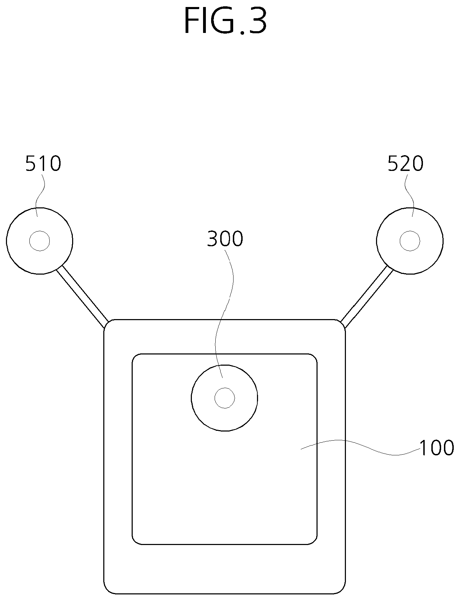

FIG. 2 is a view showing the overall appearance of a mobile robot 10 for gait analysis according to an exemplary embodiment of the present disclosure, and FIG. 3 is a top view of a mobile robot 10 for gait analysis according to an exemplary embodiment of the present disclosure. As shown in FIGS. 2 and 3, the gait measurement LiDAR sensor unit 500 of the mobile robot 10 for gait analysis according to the invention includes left LiDAR sensor unit 510 and a right LiDAR sensor unit 520.

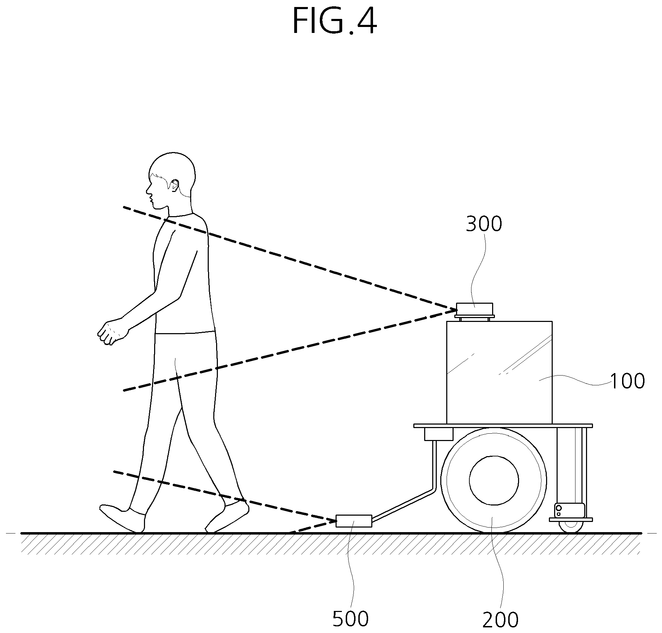

FIG. 4 is a view showing that a mobile robot 10 for gait analysis moves and scans a gait of a subject to be measured according to an exemplary embodiment of the present disclosure. As shown in FIGS. 2 to 4, the mobile robot 10 for gait analysis according to an exemplary embodiment of the present disclosure may allow the controller 400 to control the driving unit 200 to follow the subject to be measured with a preset distance maintained by using the scanning information of the front LiDAR sensor unit 300 mounted on the main body 100 such that the movement of the foot of the subject to be measured may be measured using the gait measurement LiDAR sensor unit 500 installed to protrude forward from the side of the main body 100 and may allow the gait analysis unit 600 to analyze the gait by analyzing the measured movement of the foot. Therefore gait analysis is simply and easily performed without attaching a sensor to the body of the subject to be measured and the gait is continuously analyzed without restrictions on places since tracking and measuring the subject to be measured.

A LiDAR sensor is a device that identifies the location and shape of an object through laser scanning and is applied to aircraft and autonomous vehicles. Using the LiDAR sensor, gait analysis may be performed by accurately measuring footprints, including the position, shape, direction, and form of the foot of the subject to be measured. Since the recognition rate decreases as the distance from the measurement reference point increases due to the nature of LiDAR, there may be a problem of making recognition difficult when the subject to be measured is more than 2 meters away from the LiDAR sensor especially when the size of the foot is small in the case of a footprint. Therefore, there may be a limitation in that it is difficult for a fixed LiDAR sensor to observe a footprint over a wide moving distance.

The mobile robot 10 for gait analysis according to an exemplary embodiment of the present disclosure may be configured as a mobile object, and may collect and store path and footprint measurement results while following the subject to be measured with the gait measurement LiDAR sensor unit 500 mounted. Therefore, when using the mobile robot 10 for gait analysis according to an exemplary embodiment of the present disclosure, it may be possible to continuously track and analyze the gait in various environments beyond a designated location.

Hereinafter, each configuration of the mobile robot 10 for gait analysis according to an exemplary embodiment of the present disclosure will be described in detail with reference to FIGS. 1 to 4.

The main body 100 may form the body of the mobile robot 10 for gait analysis according to an exemplary embodiment of the present disclosure and may have various configurations embedded therein, such as the controller 400, the gait analysis unit 600, and the like. In FIG. 2, the main body 100 may be composed of a plate and a box, but is not limited thereto, and the shape of the main body 100 may vary.

The driving unit 200 may move the main body 100. That is, as shown in FIG. 2, the driving unit 200 may be composed of at least one wheel or the like disposed below the main body 100 so that the mobile robot 10 for gait analysis according to an exemplary embodiment of the present disclosure is configured to be mobile.

The front LiDAR sensor unit 300 may be mounted on the main body 100 to scan the front side. More specifically, the front LiDAR sensor unit 300 may be installed at the torso height of the subject to be measured. That is, as shown in FIG. 4, the front LiDAR sensor unit 300 may scan the torso height of the subject to be measured going ahead so that the distance to the subject to be measured remains constant. In addition, the front LiDAR sensor unit 300 may be installed on the upper side of the main body 100 to scan a plane at the torso height between the knees and shoulders of the subject to be measured, thereby detecting the torso position of the subject to be measured and the direction in which the torso faces.

The controller 400 may control the driving unit 200 to follow the subject to be measured with a preset distance maintained by using the scanning information of the front LiDAR sensor unit 300. That is, the controller 400 may identify the information of the subject to be measured in real time using the position and direction of the torso of the subject measured by the front LiDAR sensor unit 300, and accordingly control the driving unit 200 to move in an appropriate speed, direction, and the like so that the mobile robot 10 for gait analysis according to an exemplary embodiment of the present disclosure maintains a certain distance from the subject to be measured.

The gait measurement LiDAR sensor unit 500 may be installed to protrude forward from the side of the main body 100 and may measure the movement of the foot of the subject to be measured. More specifically, the gait measurement LiDAR sensor unit 500 may measure the movement of the foot, including changes in the positions, directions, and shapes of the left and right feet by scanning a plane at the foot height of the subject to be measured.

Herein, the laser scanning height of the gait measurement LiDAR sensor unit 500 may be approximately 3 to 10 cm above the ground and, more specifically, may be 5 cm in height. The gait measurement LiDAR sensor unit 500 may track and observe the footprints of the gait of the subject to be measured by generating scanning data while scanning with laser beams the plane parallel to the ground at a height of 3-10 cm from the ground.

In addition, the gait measurement LiDAR sensor unit 500 according to the invention includes a left LiDAR sensor unit 510 that is installed to protrude forward from the left side of the main body 100 and measures the movement of the left foot of the subject to be measured, and a right LiDAR sensor unit 520 that is installed to protrude forward from the right side of the main body 100 and measures the movement of the right foot of the subject to be measured.

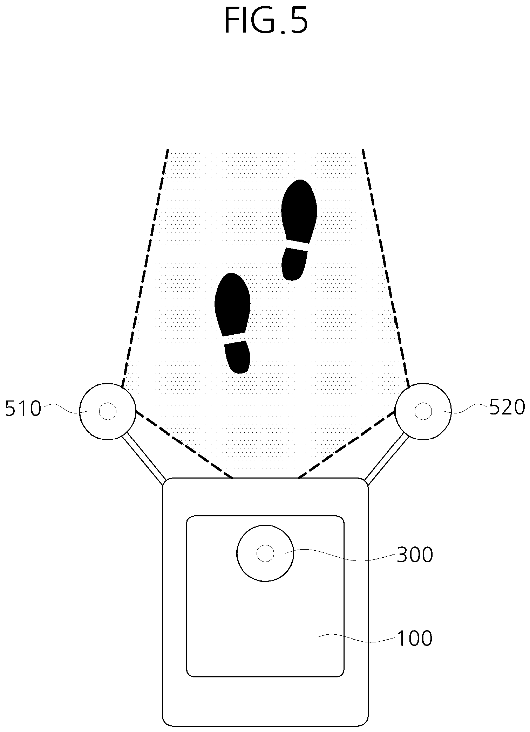

FIG. 5 is a view showing that a gait measurement LiDAR sensor unit 500 of a mobile robot 10 for gait analysis measures the movement of the foot of the subject to be measured according to an exemplary embodiment of the present disclosure. As shown in FIG. 5, the mobile robot 10 for gait analysis according to an exemplary embodiment of the present disclosure may allow the left LiDAR sensor unit 510 and the right LiDAR sensor unit 520 to be installed to protrude in the shape of a "Y" and to measure the left foot and right foot of the walking subject to be measured, respectively. In particular, since the two LiDAR sensor units 510, 520 scan the movement of the two feet of the subject to be measured, it may be possible to minimize the possibility of the measurement failure that is caused by interference by other objects or overlapping of the two feet, and to precisely measure the position, direction, shape, and the like of the foot.

The gait analysis unit 600 may analyze the gait of the subject to be measured by using the movement of the foot measured by the gait measurement LiDAR sensor unit 500. More specifically, the gait analysis unit 600 may analyze the gait of the subject to be measured by comprehensively analyzing the movement of the left foot and the right foot measured by the left LiDAR sensor unit 510 and the right LiDAR sensor unit 520 and the movement path and movement speed of the mobile robot for gait analysis 10. In FIG. 2, the gait analysis unit 600 may be mounted behind the driving unit 200, but the location of the gait analysis unit 600 may vary, such as being embedded in the main body 100. Depending on exemplary embodiments, the function of the gait analysis unit 600 may be performed in an electronic device connected to the mobile robot 10 for gait analysis through a network.

In addition, the gait analysis unit 600 may analyze at least one selected from the group, including a stride length, the number of steps, and a walking speed. First, the gait analysis unit 600 may receive the scanning data generated by the gait measurement LiDAR sensor unit 500 and recognize the foot position of the subject to be measured from the scanning data. The gait analysis unit 600 may analyze at least one selected from the group, including the stride length, the number of steps, and the walking speed using the recognized position of the foot. In addition, the left and right gait balance may be analyzed using the position of the left foot and right foot and the step distances of each of the left foot and right foot. Depending on exemplary embodiments, the gait analysis unit 600 may recognize the angle of the foot of the subject to be measured from the scanning data.

Meanwhile, the gait analysis unit 600 may predict the type of disease from the gait analysis result by using the artificial intelligence model that has been trained completely through inputting the stride length, the number of steps, the walking speed, the left and right gait balance, and the foot angle and outputting the type of disease. That is, the types of predicted diseases such as musculoskeletal diseases, vestibular abnormalities, Parkinson's disease, and dementia may be obtained as an output by inputting into the artificial intelligence model the stride length, the number of steps, the walking speed, the left and right gait balance, and the foot angle, which are the results of the gait analysis for the subject to be measured by the gait analysis unit 600. These output values may be provided to the client terminal of the medical staff to help the medical staff make decisions.

Herein, the learning algorithm of the artificial intelligence model may be any one of a random forest, a convolutional neural network (CNN), a recurrent neural network (RNN), and a transformer, and an ensemble algorithm that combines two or more algorithms may be used.

Depending on exemplary embodiments, transfer learning may be used. Transfer learning may be to reuse a pre-trained model for a new problem. Since a pre-trained model is used, there may be an advantage of being able to train a deep neural network with relatively little data. In addition, it may be useful because most real-world problems typically do not have millions of labeled data to train complex models. Using such transfer learning, the present disclosure may be possible to predict the type of disease with greater detail and accuracy by generating an artificial intelligence model for each group for the measured subject grouped by gender, age, etc. from an artificial intelligence model which is pre-trained with large amounts of data.

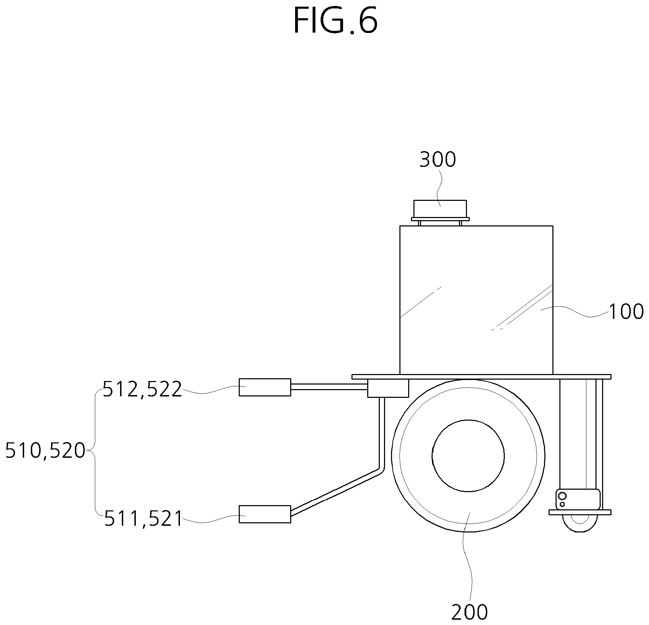

FIG. 6 is a view showing that a gait measurement LiDAR sensor unit 500 of a mobile robot 10 for gait analysis includes a first LiDAR sensor unit 511, 521 and a second LiDAR sensor unit 512, 522 according to an exemplary embodiment of the present disclosure. As shown in FIG. 6, the left LiDAR sensor unit 510 and the right LiDAR sensor unit 520 of the mobile robot 10 for gait analysis according to an exemplary embodiment of the present disclosure may include a first LiDAR sensor unit 511, 521 that scans a plane at the foot height of the subject to be measured and collects a first scanning data and a second LiDAR sensor unit 512, 522 that scans a plane at the knee height of the subject to be measured and collects a second scanning data. The gait analysis unit 600 may analyze at least one selected from the group consisting of including body distortion and walking instability when the subject to be measured walks by combining the foot position, knee position, and torso position of the subject to be measured.

That is, the left LiDAR sensor unit 510 and the right LiDAR sensor unit 520 may scan a plane at a predetermined height from the ground, and may detect different parts of the body of the subject to be measured by including the first LiDAR sensor units 511, 521 and the second LiDAR sensor units 512, 522, which scan the plane at different heights respectively.

More specifically, the first LiDAR sensor units 511, 521 may collect the first scanning data by scanning a plane at the foot height of the walking subject to be measured. That is, the first LiDAR sensor units 511, 521 may detect the foot position, foot shape, and foot direction of the subject to be measured by scanning the plane at the foot height of the subject to be measured while being attached to the floor or being installed close to the floor. The laser scanning height of the first LiDAR sensor units 511, 521 may be approximately 3 to 10 cm above the ground, more specifically, 5 cm in height.

The second LiDAR sensor units 512, 522 may collect the second scanning data by scanning the plane at the knee height of the walking subject to be measured. That is, the second LiDAR sensor units 512, 522 may be installed in the main body 100 in order to detect the knee position of the subject to be measured and the direction in which the knee is directed. The laser scanning height of the second LiDAR sensor units 512, 522 may be approximately 20 to 50 cm above the ground and may be configured to be adjustable in height.

Herein, the first LiDAR sensor units 511, 521 and the second LiDAR sensor units 512, 522 may be installed to protrude in the shape of "Y" from the main body 100 as shown in FIGS. 3 and 5. In addition, the front LiDAR sensor unit 300 may be configured to scan the pelvis height of the subject to be measured and identify pelvic distortion from the position and direction of the pelvis. The laser scanning height of the front LiDAR sensor unit 300 may be 50 to 100 cm above the ground, and may be configured to be adjustable in height.

The height adjustment unit (not shown) may be a configuration for adjusting the height of the second LiDAR sensor units 512, 522 and the front LiDAR sensor unit 300, and may be composed of a rail or the like. By adjusting the height of the second LiDAR sensor units 512, 522 and the front LiDAR sensor unit 300 to match the height of the knee, the pelvis, etc. of the subject to be measured using the height adjustment unit (not shown), it may be possible to accurately measure the gait of the subject having different heights to be measured, such as an adult or a child.

The gait analysis unit 600 may analyze at least one selected from the group consisting of body distortion and walking instability when the subject to be measured walks by combining the foot position, knee position, and torso position of the subject to be measured. First, the gait analysis unit 600 may specify the foot position of the subject to be measured by analyzing the first scanning data, the knee position of the subject to be measured by analyzing the second scanning data, and the torso position of the subject to be measured by analyzing the scanning data of the front LiDAR sensor unit 300, and may analyze at least one selected from the group, including the stride length, the number of the steps, and the walking speed by using the foot position of the subject to be measured, and analyze at least one selected from the group, including the distance between the knees and the movement speed of the knee by using the knee position of the subject to be measured.

In addition, the gait analysis unit 600 may analyze at least one selected from the group consisting of including body distortion and walking instability when the subject to be measured walks by combining the foot position, knee position, and torso position of the specified subject to be measured. In this way, by analyzing the movement patterns of the torso, the knees, and the feet when walking, it may be possible to provide information necessary for rehabilitation medicine, such as shifts in the body's center of gravity, and to be applied to the cases such as elderly people walking with a cane, individuals with scoliosis, and changes in prognosis before and after surgery.

As described above, the mobile robot 10 for gait analysis proposed by the present disclosure may perform gait analysis simply and easily without attaching a sensor to the body of the subject to be measured and may continuously analyze the gait without restrictions on places since tracking and measuring the subject to be measured, by measuring the movement of the foot of the subject to be measured and by analyzing the gait using the scanning information of the gait measurement LiDAR sensor unit 500 installed to protrude forward from the side of the main body 100 while following the subject to be measured with a preset distance maintained by using the scanning information of the front LiDAR sensor unit 300 mounted on the main body 100.

Meanwhile, the present disclosure may include a computer-readable medium including a program command for performing operations implemented in various communication terminals. For example, computer-readable media may include magnetic media such as hard disks, floppy disks and magnetic tapes, optical media such as CD-ROMs and DVDs, magneto-optical media such as floptical disks, and hardware devices specifically configured to store and execute program commands such as ROMs, RAMs, flash memories, etc.

Such computer-readable media may include program instructions, data files, data structures, and the like, alone or in combination. In this case, a program command recorded on computer-readable media may be specifically designed and configured to implement the present disclosure or may be known and available to those skilled in the art of computer software. For example, it may include not only machine language codes such as those created by a compiler, but also advanced language codes that may be executed by a computer using an interpreter or the like.

The description of the present disclosure described above is for illustrative purposes only, and those skilled in the art to which the present disclosure belongs will understand that the present disclosure can be easily modified into other specific forms without changing the technical idea or essential features of the present disclosure. Therefore, it should be understood that the exemplary embodiments described above are exemplary in all respects and not limited. For example, each component described in a single form may be implemented in a distributed manner, and similarly, components described as distributed may also be implemented in a combined form.

The scope of the present disclosure is represented by the claims described below rather than the detailed description above, and all modifications or variations derived from the equivalents thereof shall be construed as being included in the scope of the present disclosure.

## Claims

1. A mobile robot (10) for gait analysis, the robot comprising:
a main body (100);
a driving unit (200) for moving the main body (100);
a front LiDAR sensor unit (300) being mounted on the main body (100) and scanning a front side;
a controller (400) for controlling the driving unit (200) to follow a subject to be measured with a preset distance maintained, by using scanning information of the front LiDAR sensor unit (300);
a gait measurement LiDAR sensor unit (500) being installed to protrude forward from a side of the main body (100) and measuring movements of feet of the subject to be measured; and
a gait analysis unit (600) for analyzing a gait of the subject to be measured, by using the movements of the feet measured by the gait measurement LiDAR sensor unit (500),
wherein the gait measurement LiDAR sensor unit (500) measures the movements of the feet, including changes in position, direction, and shape of left and right feet, by scanning a plane at a foot height of the subject to be measured, and
**characterized in that** the gait measurement LiDAR sensor unit (500) comprises:
a left LiDAR sensor unit (510) being installed to protrude forward from a left side of the main body (100) and measuring the movement of a left foot of the subject to be measured; and
a right LiDAR sensor unit (520) being installed to protrude forward from a right side of the main body (100) and measuring the movement of a right foot of the subject to be measured.

2. The robot of claim 1, wherein the front LiDAR sensor unit (300) is installed at a torso height of the subject to be measured.

3. The robot of claim 1, wherein the gait analysis unit (600) analyzes the gait of the subject to be measured by comprehensively analyzing
the movement of the left foot and right foot measured by the left LiDAR sensor unit (510) and the right LiDAR sensor unit (520), and
a movement path and a movement speed of the mobile robot (10) for gait analysis.

4. The robot of claim 1, wherein each of the left LiDAR sensor unit (510) and the right LiDAR sensor unit (520) comprises:
a first LiDAR sensor unit configured to collect a first scanning data by scanning the plane at the foot height of the subject to be measured; and
a second LiDAR sensor unit configured to collect a second scanning data by scanning a plane at a knee height of the subject to be measured.

5. The robot of claim 4, wherein the gait analysis unit (600) analyzes at least one selected from a group consisting of body distortion and gait instability when the subject to be measured walks, by combining foot position, knee position, and torso position of the subject to be measured.

## Patentansprüche

1. Mobiler Roboter (10) zur Ganganalyse, wobei der Roboter umfasst:
einen Hauptkörper (100);
eine Antriebseinheit (200) zum Bewegen des Hauptkörpers (100);
eine Front-LiDAR-Sensoreinheit (300), die am Hauptkörper (100) montiert ist und eine Vorderseite abtastet;
eine Steuereinheit (400) zum Steuern der Antriebseinheit (200), um einer zu messenden Person unter Aufrechterhaltung eines voreingestellten Abstands zu folgen, unter Verwendung von Abtastinformationen der Front-LiDAR-Sensoreinheit (300);
eine Gangmessungs-LiDAR-Sensoreinheit (500), die von einer Seite des Hauptkörpers (100) nach vorne vorstehend installiert ist und Bewegungen der Füße der zu messenden Person misst; und
eine Ganganalyseeinheit (600) zum Analysieren eines Gangs der zu messenden Person unter Verwendung der von der Gangmessungs-LiDAR-Sensoreinheit (500) gemessenen Bewegungen der Füße,
wobei die Gangmessungs-LiDAR-Sensoreinheit (500) die Bewegungen der Füße, einschließlich Änderungen der Position, der Richtung und der Form des linken und des rechten Fußes, durch Abtasten einer Ebene in einer Fußhöhe der zu messenden Person misst,
**dadurch gekennzeichnet, dass** die Gangmessungs-LiDAR-Sensoreinheit (500) umfasst:
eine linke LiDAR-Sensoreinheit (510), die von einer linken Seite des Hauptkörpers (100) nach vorne vorstehend installiert ist und die Bewegung eines linken Fußes der zu messenden Person misst; und
eine rechte LiDAR-Sensoreinheit (520), die von einer rechten Seite des Hauptkörpers (100) nach vorne vorstehend installiert ist und die Bewegung eines rechten Fußes der zu messenden Person misst.

2. Roboter nach Anspruch 1, wobei die Front-LiDAR-Sensoreinheit (300) in einer Rumpfhöhe der zu messenden Person installiert ist.

3. Roboter nach Anspruch 1, wobei die Ganganalyseeinheit (600) den Gang der zu messenden Person durch umfassendes Analysieren
der von der linken LiDAR-Sensoreinheit (510) und der rechten LiDAR-Sensoreinheit (520) gemessenen Bewegung des linken und des rechten Fußes und
einer Bewegungsbahn und einer Bewegungsgeschwindigkeit des mobilen Roboters (10) zur Ganganalyse analysiert.

4. Roboter nach Anspruch 1, wobei jede der linken LiDAR-Sensoreinheit (510) und der rechten LiDAR-Sensoreinheit (520) umfasst:
eine erste LiDAR-Sensoreinheit, die dazu konfiguriert ist, erste Abtastdaten durch Abtasten der Ebene in der Fußhöhe der zu messenden Person zu erfassen; und
eine zweite LiDAR-Sensoreinheit, die dazu konfiguriert ist, zweite Abtastdaten durch Abtasten einer Ebene in einer Kniehöhe der zu messenden Person zu erfassen.

5. Roboter nach Anspruch 4, wobei die Ganganalyseeinheit (600) wenigstens eines, ausgewählt aus einer Gruppe bestehend aus Körperverzerrung und Ganginstabilität, wenn die zu messende Person geht, durch Kombinieren einer Fußposition, einer Knieposition und einer Rumpfposition der zu messenden Person analysiert.

## Revendications

1. Robot mobile (10) pour l'analyse de la démarche, le robot comprenant :
un corps principal (100) ;
une unité d'entraînement (200) destinée à déplacer le corps principal (100) ;
une unité de capteur LiDAR avant (300), montée sur le corps principal (100) et balayant une zone à l'avant ;
un dispositif de commande (400) destiné à commander l'unité d'entraînement (200) afin de suivre un sujet faisant l'objet de mesures tout en maintenant une distance prédéfinie, au moyen d'informations de balayage de l'unité de capteur LiDAR avant (300) ;
une unité de capteur LiDAR de mesure de la démarche (500), installée de manière à faire saillie vers l'avant depuis un côté du corps principal (100) et mesurant les mouvements des pieds du sujet faisant l'objet de mesures ; et
une unité d'analyse de la démarche (600) destinée à analyser une démarche du sujet faisant l'objet de mesures, au moyen des mouvements des pieds mesurés par l'unité de capteur LiDAR de mesure de la démarche (500),
dans lequel l'unité de capteur LiDAR de mesure de la démarche (500) mesure les mouvements des pieds, comprenant des changements de position, de direction et de forme du pied gauche et du pied droit, en balayant un plan à la hauteur des pieds du sujet faisant l'objet de mesures, et
**caractérisé en ce que** l'unité de capteur LiDAR de mesure de la démarche (500) comprend :
une unité de capteur LiDAR gauche (510), installée de manière à faire saillie vers l'avant depuis un côté gauche du corps principal (100) et mesurant le mouvement d'un pied gauche du sujet faisant l'objet de mesures ; et
une unité de capteur LiDAR droite (520), installée de manière à faire saillie vers l'avant depuis un côté droit du corps principal (100) et mesurant le mouvement d'un pied droit du sujet faisant l'objet de mesures.

2. Robot selon la revendication 1, dans lequel l'unité de capteur LiDAR avant (300) est installée à la hauteur du torse du sujet faisant l'objet de mesures.

3. Robot selon la revendication 1, dans lequel l'unité d'analyse de la démarche (600) analyse la démarche du sujet faisant l'objet de mesures en analysant de manière globale
le mouvement du pied gauche et du pied droit mesuré par l'unité de capteur LiDAR gauche (510) et l'unité de capteur LiDAR droite (520), et
une trajectoire de déplacement et une vitesse de déplacement du robot mobile (10) pour l'analyse de la démarche.

4. Robot selon la revendication 1, dans lequel l'unité de capteur LiDAR gauche (510) et l'unité de capteur LiDAR droite (520) comprennent chacune :
une première unité de capteur LiDAR configurée pour recueillir de premières données de balayage en balayant le plan à la hauteur des pieds du sujet faisant l'objet de mesures ; et
une deuxième unité de capteur LiDAR configurée pour recueillir de deuxièmes données de balayage en balayant un plan à la hauteur des genoux du sujet faisant l'objet de mesures.

5. Robot selon la revendication 4, dans lequel l'unité d'analyse de la démarche (600) analyse au moins un élément sélectionné parmi un groupe constitué d'une distorsion du corps et d'une instabilité de la démarche lorsque le sujet faisant l'objet de mesures marche, en combinant la position du pied, la position du genou et la position du torse du sujet faisant l'objet de mesures.
